# EUROPÄISCHE PATENTANMELDUNG

(11) **EP 1 775 281 A1**
(43) Veröffentlichungstag der Anmeldung: **18.04.2007**
(21) Anmeldenummer: 06019315.8
(22) Anmeldetag: 15.09.2006
(51) Int. Cl.: C07C 209/26, C07C 211/27, C07C 211/29

(54) **Verfahren zur reduktiven Aminierung von Ketonen und Aldehyden mit wässrigen Aminen**

(30) Priorität: 01.10.2005 DE 102005047288
(71) Anmelder: Clariant Speciality Fine Chemicals (Deutschland) GmbH, 65929 Frankfurt am Main (DE)
(72) Erfinder: Schramm, Daniel, Dr., 65843 Sulzbach (DE); Decker, Daniel, Dr., 65835 Liederbach a. Ts. (DE); Forstinger, Klaus, Dr., 64832 Babenhausen (DE); Flick, Klemens, Prof.Dr., 74080 Heilbronn (DE)
(74) Vertreter: Mikulecky, Klaus

(57) **Zusammenfassung**

Verfahren zur Herstellung von Aminen der allgemeinen Formel (I)

R¹R²CHNR³R⁴ (I)

in der R¹, R², R³ und R⁴ unabhängig voneinander Wasserstoff, geradkettiges oder verzweigtes, halogeniertes oder halogenfreies, C₁-C₁₂-Alkyl, C₃-C₁₂-Cycloalkyl, C₆-C₁₀-Aryl oder C₇-C₁₁-Aralkyl bedeuten, durch katalytische, hydrierende Aminierung von Carbonylverbindungen der allgemeinen Formel (II)

R¹-C(=O)-R² (II)

mit Stickstoffverbindungen der allgemeinen Formel (III)

HNR³R⁴ (III)

in Gegenwart von bifunktionalen Katalysatorsystemen, enthaltend a.) eine hydrieraktive Katalysatorkomponente, enthaltend ein oder mehrere Metalle der 8. Nebengruppe des Periodensystemsund b.) einen oder mehrere feste, saure Cokatalysatoren, wobei die hydrierende Aminierung in Gegenwart von wässrigem Ammoniak oder wässrigen Amin der Formel (III) und mindestens einem mit Ammoniak oder dem wässrigen Amin der Formel (III) mischbaren organischen Lösungsmittel durchgeführt wird.

## Beschreibung

Die reduktive Aminierung von Ketonen und Aldehyden ist eine in der Technik übliche Methode zur Herstellung von Aminen aus Aldehyden bzw. Ketonen. Die Umsetzung wird großtechnisch immer in einem Überschuss von wasserfreiem Ammoniak durchgeführt, um die Bildung von sekundären Aminen zu verhindern und die Bildung des intermediär auftretenden Imins zu erleichtern. Das Imin bildet sich durch eine Addition von Ammoniak an die Carbonylgruppe und anschließende Eliminierung von Wasser. In wässrigen Medien liegt das Gleichgewicht der Eliminierungsreaktion weit auf der Seite der Edukte.

Die Reaktion wird üblicherweise in Gegenwart von metallischen Katalysatoren durchgeführt. Dabei werden beispielsweise Raney-Ni, Raney-Co, Pt/C, Pd/C, Pd/BaSO₄ oder Rh/Al₂O₃ als Katalysatoren eingesetzt. In Houben-Weyl, Methoden der Organischen Chemie, 4. Aufl., Bd. IV/1c, Reduktion Teil 1, Thieme Verlag, 1980, Seiten 436-437 wird angegeben, dass Halogenaromaten bei der hydrierenden Aminierung mit Raney-Nickel nicht angegriffen werden. Es werden zwei beispielhafte Umsetzungen angegeben, die jedoch zu geringen Ausbeuten führen. Vermutlich trat bei den angegeben Beispielen eine Dehalogenierung als wesentliche Nebenreaktion auf.

GB-A- 969 977 beschreibt substituierte 1,3-Diphenyl-propylamine und entsprechende lmine. Im Beispiel 6 wird die hydrierenden Aminierung von 1-(2-Ethyl-4,5-dimethoxyphenyl)-3-(4-chlorphenyl)-1-propenol mit Ammoniak an Raney-Ni als Katalysator beschrieben. Nach der Aufarbeitung wird 1-Amino-1-(2-ethyl-4,5-dimethoxyphenyl)-3-(chlorphenyl) propanhydrochlorid als Produkt erhalten.

EP-A-355 351 betrifft ein Verfahren zur Herstellung von Aminen durch reduktive Aminierung von Oxo-Verbindungen, die halogensubstituierte aromatische Substituenten enthalten. Die Umsetzung wird insbesondere an Raney-Ni oder Raney-Co in Gegenwart von Ammoniak oder Aminen durchgeführt. Zudem wird die Umsetzung in Gegenwart von organischen Schwefelverbindungen wie Dimethylsulfoxid oder Bis-(2-hydroxyethyl)-sulfid durchgeführt. Die organische Schwefelverbindung wird in Mengen von vorzugsweise 1 bis 25 Gew.-%, bezogen auf den Katalysator, eingesetzt. In Bezug auf die Carbonylverbindung liegen Mengen von mehr als 1 Gew.-% vor. Die Schwefelverbindungen dienen zur partiellen Vergiftung des Katalysators, so dass das aromatisch gebundene Halogen erhalten werden kann. Beispielsweise werden bei der Aminierung von p-Chloracetophenon unter geeigneten Bedingungen Ausbeuten an p-Chlorphenylethylamin von bis zu 90 % erzielt.

Der Zusatz der relativ großen Mengen an Schwefelverbindungen ist jedoch insofern nachteilig, da eine weitere Komponente in das Reaktionssystem eingeführt wird, die die Aufarbeitung der Reaktionsprodukte insbesondere durch Destillation, erschweren kann. Zudem verursachen die organischen Schwefelverbindungen einen höheren Katalysatorverbrauch, der typischerweise über 6 Gew.-% des Ni-Katalysators, bezogen auf die eingesetzten Carbonylverbindungen, beträgt.

EP-A-1201642 beschreibt daher ein Verfahren zur Herstellung von Aminen aus halogenierten Aromaten mit wasserfreiem Ammoniak. Um eine Dehalogenierung des Aromaten zu verhindern wird ein fester saurer Cokatalysator zugesetzt. Im Beispiel 6 wird dabei p-Choracetophenon mit 1,6 Gew.-% Raney-Co und 19 Gew.-% Zr02, bezüglich des Edukts, in Methanol mir Ammoniak zu p-Chlorphenylethylamin in > 98 %iger Ausbeute umgesetzt.

Das Arbeiten in wasserfreien Systemen, wie es im Stand der Technik beschrieben wird, bedeutet jedoch einen erheblichen Verfahrensaufwand, der insbesondere bei einer großtechnischen Herstellung das Verfahren unwirtschaftlich macht.

Die Aufgabe der vorliegenden Erfindung ist daher die Bereitstellung eines Verfahrens zur Herstellung von Aminen durch reduktive Aminierung von Carbonylverbindungen in wässrigen Ammoniaklösungen, dass die Nachteile der bekannten Verfahren vermeidet und in hohen Ausbeuten mit guter Selektivität bei einfacher Verfahrensweise zu den gewünschten Aminen führt.

Die Aufgabe wird erfindungsgemäß gelöst durch ein Verfahren zur Herstellung von Aminen der allgemeinen Formel (I)

R¹R²CHNR³R⁴ (I)

in der R¹, R², R³ und R⁴ unabhängig voneinander Wasserstoff, geradkettiges oder verzweigtes C₁ - C₁₂- Alkyl, C₃-C₁₂-Cycloalkyl, C₆-C₁₀- Aryl oder C₇-C₁₁- Aralkyl bedeuten, durch katalytische, hydrierende Aminierung von Carbonylverbindungen der allgemeinen Formel (II)

R¹-C(=O)-R² (II)

mit Stickstoffverbindungen der allgemeinen Formel (III)

HNR³R⁴ (III)

in Gegenwart von bifunktionalen Katalysatorsystemen, enthaltend a.) eine hydrieraktive Katalysatorkomponente, enthaltend ein oder mehrere Metalle der 8. Nebengruppe des Periodensystems und b.) einen oder mehrere feste, saure Cokatalysatoren, dadurch gekennzeichnet, dass die hydrierende Aminierung in Gegenwart von wässrigem Ammoniak und mindestens einem mit Ammoniak mischbaren organischen Lösungsmittel durchgeführt wird.

Überraschenderweise hat es sich nun gezeigt, dass Carbonylverbindungen, insbesondere Ketone und Aldehyde in wässrigen Reaktionslösungen enthaltend eine oder mehrere Stickstoffverbindungen, insbesondere Ammoniak, in einer Konzentration von 0,1 bis 80 Gew.-%, insbesondere 1 bis 25 Gew.-% und einem damit mischbaren organischen Lösungsmittel in Gegenwart von Wasserstoff zu Aminen umgesetzt werden können.

Als Lösungsmittel für das erfindungsgemäße Verfahren kommen polar protische Lösungsmittel, wie z.B. Alkohole, wie Methanol, Ethanol, Propanol oder Butanol, oder auch Wasser oder Mischungen derselben untereinander zum Einsatz. Der Lösungsmittelgehalt der Reaktionsmischungen liegt vorzugsweise im Bereich von 20 bis 85, insbesondere 30 bis 70 Gew.-%.

Erfindungsgemäß einsetzbare Carbonylverbindungen der Formel (II) sind zum Beispiel Acetophenon, Methylacetophenon, F-Acetophenon, Benzaldehyd und F-Benzaldehyd, 4,4'-Dichlor-bezophenon, um nur einige zu nennen.
Als Stickstoffverbindungen der Formel (III) werden vorzugsweise Amine ausgewählt aus folgender Gruppe eingesetzt: Ammoniak, Methylamin, Ethylamin, Propylamin, Dimethylamin, Diethylamin, Dipropylamin. Vorzugsweise wird Ammoniak verwendet. Die Stickstoffverbindungen der Formel (III) können gleichzeitig auch als Lösungsmittel dienen.

Die Geschwindigkeit und Selektivität der Reaktion zum gewünschten Amin wird durch die Verwendung eines bifunktionellen Katalysatorsystems (z.B. Pd/TiO₂ oder Pd/C + TiO₂) erhöht. Das Katalysatorsystem setzt sich zusammen aus einem oder mehreren festen, sauren Cokatalysatoren, die ein oxidisches Material enthalten, das in der Lage ist die lminbildung zu katalysieren und einer hydrieraktiven Katalysatorkomponente.

Als Cokatalysator können insbesondere saure Oxide wie Al₂O₃, TiO₂, SiO₂, Cr₂O₃, ZrO₂, insbesondere TiO₂, ZrO₂ oder deren Mischungen eingesetzt werden.
Die hydrieraktive Komponente enthält ein oder mehrere Elemente der Übergangsmetalle der 8. Nebengruppe des Periodensystems. Bevorzugt werden Pd, Pt, Co, Ni, Ru und Rh oder deren Verbindungen verwendet. Die Metalle der hydrieraktiven Komponente können auch in beliebigen Gewichtsverhältnissen vermischt eingesetzt werden und zusätzlich weitere Metalle, wie z.B. Fe, Mo, Cu, Ag, V, Zn, W in Anteilen von 0 bis 5 Gew.-% enthalten. In dem erfindungsgemäßen Verfahren werden die Katalysatoren vorzugsweise in Form von elementarem Metall, in Form der Oxide oder auf Trägern gebunden eingesetzt. Beispiele hierfür sind Raney-Cobalt oder Raney-Nickel, als Metall Schwamm, elementares Pd, Pt, Rh, Ru, Ir auf Trägern, wie z.B. C, Al₂O₃, SiO₂, TiO₂, ZrO₂, BaSO₄, MgO, Cr₂O₃.

Die hydrieraktive Komponente wird in einer Menge von 0,1 bis 10 Gew.-%, bevorzugt 1 bis 5 Gew. % bezogen auf die zu aminierende Carbonylverbindung eingesetzt.

Der Cokatalysator kann als Träger für die hydrieraktive Komponente verwendet werden, ebenso ist es möglich beide Katalysatorkomponenten in Form einer Suspension einzusetzen.

Die Aminierung wird vorzugsweise bei Temperaturen im Bereich von 30 bis 200°C, insbesondere bei Temperaturen im Bereich von 50 bis 180°C durchgeführt, bei einem Wasserstoffdruck von 1 bis 100 bar, insbesondere 2 bis 80 bar.

Das erfindungsgemäße Verfahren wird bevorzugt im Autoklaven durchgeführt, die Hydrierung kann aber auch im Festbett erfolgen.

Das erfindungsgemäße Verfahren zeichnet sich durch eine einfache und wirtschaftliche Verfahrensführung aus und erlaubt es gleichzeitig die gewünschten Aminverbindungen mit hohen Selektivitäten und guten Ausbeuten herzustellen.

### Beispiele

### Beispiel 1 (V32)

### Umsetzung von Methylacetophenon zum Amin an Pd/C und Ti02

10 ml Methylacetophenon wurden in einem Autoklaven (Fa. Berghoff) mit 30 ml konzentrierter wässriger Ammoniaklösung (25 Gew.-%), 40 ml Methanol und 500 mg eines handelsüblichen Pd/C-Katalysators (5 % Pd auf Aktivkohle, Fa. AMC) und 500 mg Ti02 (VKR611, Fa. Sachtleben) gemischt. Die Reaktionsmischung wurde auf eine Temperatur von 84°C erhitzt, dann Wasserstoff bis zu einem Gesamtdruck von 5,5 bar aufgepresst. Nach einer Reaktionszeit von 180 min wurde abgekühlt, entspannt und das Reaktionsgemisch gaschromatographisch untersucht. Methylacetophenon war zu 93,5 % umgesetzt. Die Selektivität zum Amin betrug 99,5 %.

### Beispiel 2 (V59)

### Umsetzung von Methylacetophenon zum Amin an Pd/Ti02

10 ml Methylacetophenon wurden in einem Autoklaven (Fa. Berghoff) mit 30 ml konzentrierter wässriger Ammoniaklösung (25 Gew.-%), 30 ml Methanol und 1000 mg eines durch Tränken von Titandioxid-Pulver (VKR611, Fa. Sachtleben) mit wässriger Pd-Nitrat-Lösung hergestellten Katalysators gemischt. Der Katalysator hatte einen Pd-Gehalt von 3 Gew.-%. Die Reaktionsmischung wurde auf eine Temperatur von 93°C erhitzt, dann Wasserstoff bis zu einem Gesamtdruck von 7 bar aufgepresst. Nach einer Reaktionszeit von 180 min wurde abgekühlt, entspannt und das Reaktionsgemisch gaschromatographisch untersucht. Methylacetophenon war zu 94 % umgesetzt. Die Selektivität zum Amin betrug 94 %.

### Beispiel 3

### Umsetzung von Acetophenon zum Amin mit wässrigem Ammoniak an Pd/C+Ti02

10 ml Acetophenon wurden in einem Autoklaven (Fa. Berghoff) mit 30 ml konzentrierter wässriger Ammoniaklösung (25 Gew.-%), 30 ml Methanol und 500 mg eines handelsüblichen Pd/C-Katalysators (5 % Pd auf Aktivkohle, Fa. AMC) und 500 mg TiO₂ (VKR611, Fa. Sachtleben) gemischt. Die Reaktionsmischung wurde auf eine Temperatur von 85°C erhitzt, dann Wasserstoff bis zu einem Gesamtdruck von 5 bar aufgepresst. Nach einer Reaktionszeit von 3 h wurde abgekühlt, entspannt und das Reaktionsgemisch gaschromatographisch untersucht. Acetophenon war zu 78 % umgesetzt. Die Selektivität zum entsprechenden Amin betrug 97 %.

### Beispiel 4

### F-Benzaldehyd mit Ni/Träger

15,3 g Ni/C-Katalysator (Kataleuna 6504 K) werden in einem 2 I Stahlautoklaven in 919 g wässriger Ammoniaklösung (27 %) suspendiert und mit 302,5 g 4-Fluorbenzaldehyd versetzt. Nach dem Erhitzen auf 120 °C wird Wasserstoff aufgepresst, wobei der Druck durch mehrfaches Zudosieren von Wasserstoff auf 38 - 45 bar gehalten wird. Nach 2,5 h verlangsamt sich die Wasserstoffaufnahme. Die Reaktion wird bei 120°C und einem Anfangsdruck von 45 bar 15 h vervollständigt. Der Autoklav wird entspannt und das Reaktionsgemisch gaschromatographisch untersucht. Zusammensetzung der Rohlösung (durch CH₂Cl₂ extrahierbarer Anteil, wasserfrei, GC [a/a]): 4-Fluorbenzylamin 90,7 %; Benzylamin 0,4 %, 4-Fluorbenzylalkohol 1,2 %; Di(4-Fluorbenzyl)amin 1,8 %; Trimer (M=352) 1,4 %, andere 4,5 %.

### Vergleichsbeispiel (V22)

### Hydrierung von Methylacetophenon mit wässrigem Ammoniak an Pd/C ohne Zusatz von TiO₂

10 ml Methylacetophenon werden in einem Autoklaven (Fa. Berghoff) mit 30 ml konzentrierter wässriger Ammoniaklösung (25 Gew.-%), 20 ml Methanol und 500 mg eines handelsüblichen Pd/C-Katalysators (5 % Pd auf Aktivkohle, Fa. AMC gemischt. Die Reaktionsmischung wird auf eine Temperatur von 99°C erhitzt, dann Wasserstoff bis zu einem Gesamtdruck von 9,4 bar aufgepresst. Nach einer Reaktionszeit von 70 min wird abgekühlt, entspannt und das Reaktionsgemisch gaschromatographisch untersucht. Methylacetophenon war zu 99 % umgesetzt. Die Selektivität zum Amin betrug jedoch nur 17,7 %.

## Patentansprüche

1. Verfahren zur Herstellung von Aminen der allgemeinen Formel (I)
R¹R²CHNR³R⁴ (I)
in der R¹, R², R³ und R⁴ unabhängig voneinander Wasserstoff, geradkettiges oder verzweigtes, halogeniertes oder halogenfreies, C₁-C₁₂-Alkyl, C₃-C₁₂-Cycloalkyl, C₆-C₁₀-Aryl oder C₇-C₁₁-Aralkyl bedeuten, durch katalytische, hydrierende Aminierung von Carbonylverbindungen der allgemeinen Formel (II)
R¹-C(=O)-R² (II)
mit Stickstoffverbindungen der allgemeinen Formel (III)
HNR³R⁴ (III)
in Gegenwart von bifunktionalen Katalysatorsystemen, enthaltend a.) eine hydrieraktive Katalysatorkomponente, enthaltend ein oder mehrere Metalle der 8. Nebengruppe des Periodensystemsund b.) einen oder mehrere feste, saure Cokatalysatoren, **dadurch gekennzeichnet, dass** die hydrierende Aminierung in Gegenwart von wässrigem Ammoniak oder wässrigen Amin der Formel (III) und mindestens einem mit Ammoniak oder dem wässrigen Amin der Formel (III) mischbaren organischen Lösungsmittel durchgeführt wird.

2. Verfahren nach Anspruch 1, **dadurch gekennzeichnet, dass** die Aminkonzentration der wässrigen Lösung im Bereich von 0,1 bis 80 Gew. %, vorzugsweise im Bereich von 1 bis 25 Gew.-% liegt.

3. Verfahren nach mindestens einem der vorhergehenden Ansprüche, **dadurch gekennzeichnet, dass** die Metalle der 8. Nebengruppe in der hydrieraktiven Katalysatorkomponente ausgewählt sind aus der Gruppe: Pd, Pt, Ru, Rh, Co, Ir und/oder Ni.

4. Verfahren nach mindestens einem der vorhergehenden Ansprüche, **dadurch gekennzeichnet, dass** die festen sauren Cokatalysatoren ausgewählt sind aus sauren Metalloxiden oder Metallmischoxiden, die in der Lage sind die Iminbildung zu katalysieren, ausgewählt aus der Gruppe Al₂O₃, TiO₂, SiO₂, Cr₂O₃, ZrO₂, insbesondere TiO₂, ZrO₂.

5. Verfahren nach mindestens einem der vorhergehenden Ansprüche, **dadurch gekennzeichnet, dass** der Cokatalysator als Träger der zweiten hydrieraktiven Katalysatorkomponente eingesetzt wird.

6. Verfahren nach mindestens einem der vorhergehenden Ansprüche, **dadurch gekennzeichnet, dass** die hydrieraktive Katalysatorkomponente und der Cokatalysator in Suspension eingesetzt wird.

7. Verfahren nach mindestens einem der vorhergehenden Ansprüche, **dadurch gekennzeichnet, dass** als Carbonylverbindungen Methylacetophenon, Fluoracetophenon, Benzaldehyd, Fluorbenzaldehyd oder 4,4'-Dichlor-benzophenon eingesetzt wird.

8. Verfahren nach mindestens einem der vorhergehenden Ansprüche, **dadurch gekennzeichnet, dass** als Stickstoffverbindung Methylamin, Ethylamin, Propylamin, Dimethylamin, Diethylamin, Dipropylamin und/ oder Ammoniak, insbesondere Ammoniak eingesetzt wird.

9. Verfahren nach mindestens einem der vorhergehenden Ansprüche, **dadurch gekennzeichnet, dass** der Lösungsmittelgehalt der Reaktionsmischung im Bereich 20 von 85 Gew.-% liegt.

10. Verfahren nach mindestens einem der vorhergehenden Ansprüche, **dadurch gekennzeichnet, dass** das mit Amin mischbare organische Lösungsmittel mindestens ein polar protisches Lösungsmittel, insbesondere ein Alkohol und/oder Wasser ist.
